# EUROPEAN PATENT APPLICATION

(11) **EP 4 810 049 A1**
(43) Date of publication of application: **23.09.2026**
(21) Application number: 26153141.2
(22) Date of filing: 21.01.2026
(51) Int. Cl.: A61K 31/737, A61P 17/00

(54) **USE OF SULFATED GLUCO-GALACTOOLIGOSACCHARIDE IN PREPARATION OF DRUGS FOR TREATING ATOPIC DERMATITIS**

(30) Priority: 20.03.2025 CN 202510334582
(71) Applicant: Nanjing University of Science and Technology, Nanjing, Jiangsu 210094 (CN)
(72) Inventor: ZHANG, Jianfa, Nanjing City, 210094 (CN); CAI, Jinpeng, Nanjing City, 210094 (CN); XU, Xi, Nanjing City, 210094 (CN)
(74) Representative: Plougmann Vingtoft a/s

(57) **Abstract**

Use of sulfated gluco-galactooligosaccharide in the preparation of drugs for treating atopic dermatitis. Sulfated gluco-galactooligosaccharide may significantly improve the skin condition of DNCB-induced atopic dermatitis mouse model, including reducing the levels of skin inflammatory cytokines, improving skin barrier function, decreasing lymph node weight and lymphocyte count. Meanwhile, the sulfated gluco-galactooligosaccharide shows no adverse effects on routine blood parameters, and possesses advantages such as low administration dosage, low pharmaceutical cost, high safety without side effects and strong efficacy, thus holding broad application prospects.

## Description

### TECHNICAL FIELD

The present disclosure belongs to the technical field of biomedicine, and relates to use of sulfated gluco-galactooligosaccharide in the preparation of drugs for treating atopic dermatitis.

### BACKGROUND

Atopic dermatitis is a multifactorial skin disease characterized by chronic and recurrent inflammatory symptoms. The clinical features of atopic dermatitis include intense pruritus and inflammatory, ulcerative lesions on the patient's skin, with pruritus and possible acute, subacute or chronic lesions as the main manifestations. In addition, the onset of atopic dermatitis is often accompanied by elevated serum immunoglobulin levels and the infiltration of immune cells such as mast cells, eosinophils and lymphocytes in the skin. Atopic dermatitis may increase the risk of developing depression, anxiety disorders, behavioral disorders, and attention deficit/hyperactivity disorder. In industrialized countries, the prevalence of atopic dermatitis is relatively high, reaching up to 20% in children and 10% in adults.

At present, three main categories of drugs are clinically used for the treatment of atopic dermatitis: first, corticosteroids, which have significant side effects including skin atrophy, telangiectasia, striae, hypopigmentation, perioral dermatitis, etc.; second, calcineurin inhibitors, which also have potential side effects including allergic responses, liver damage, nephrotoxicity, hyperuricemia and skin infections; and third, antihistamines, which, although they may relieve the pruritus caused by atopic dermatitis, have limited therapeutic effects on the disease itself. Therefore, the development of drugs for the treatment of atopic dermatitis to provide more options for personalized treatment of patients with atopic dermatitis in the future has important practical significance.

Prior studies by the inventors have reported sulfated gluco-galactooligosaccharide (SRO) and its preparation method, and have indicated that the sulfated gluco-galactooligosaccharide has anticoagulant activity (Yu N, Fang R, Ding Z, et al., Preparation and Structural Characterization of a Sulfated Octasaccharide with Heparin-like Anticoagulant Activity [J]. Carbohydrate Polymers, 2025, 347:122782). Patent application CN 114533756 A also discloses the preparation method of the sulfated gluco-galactooligosaccharide and anticoagulant activity thereof. However, whether the sulfated gluco-galactooligosaccharide exerts a therapeutic effect on atopic dermatitis has not been reported yet.

### SUMMARY

An object of the present disclosure is to provide use of sulfated gluco-galactooligosaccharide in the preparation of drugs for treating atopic dermatitis.

The sulfated gluco-galactooligosaccharide described in the present disclosure has the structural formula: 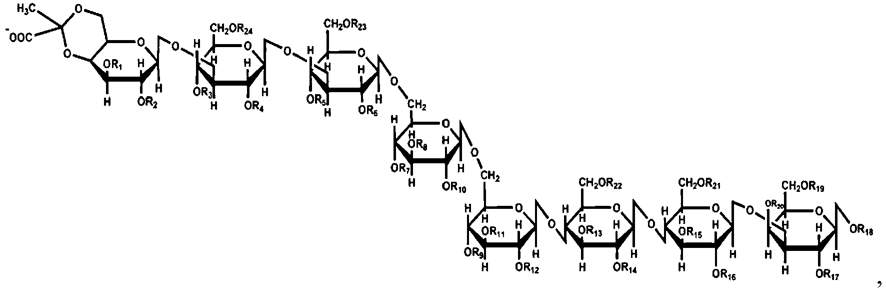 wherein, R₁, R₂, R₃, R₄, R₅, R₆, R₇, R₈, R₉, R₁₀, R₁₁, R₁₂, R₁₃, R₁₄, R₁₅, R₁₆, R₁₇, R₁₈, R₁₉, R₂₀, R₂₁, R₂₂, R₂₃ and R₂₄ are independently SO₃⁻ or H, but are not all H.

In the sulfated gluco-galactooligosaccharide of the present disclosure, the average number of sulfate groups per sugar residue is 0.5-3. In a specific embodiment of the present disclosure, an example is provided by the sulfated gluco-galactooligosaccharide having an average number of sulfate groups per sugar residue of 1.8.

A dosage form of the drug for treating atopic dermatitis in the present disclosure includes, but is not limited to, a topical solution or a cream.

The drug for treating atopic dermatitis of the present disclosure is administered by topical application.

The drug for treating atopic dermatitis of the present disclosure may be administered to any animal that may develop or has developed atopic dermatitis. These animals include human and non-human animals, such as pets or livestock.

The administration dosage of the drug for treating atopic dermatitis of the present disclosure depends on the factors such as age, health condition, body weight, and frequency of administration of the recipient.

A concentration of the sulfated gluco-galactooligosaccharide in the drug for treating atopic dermatitis of the present disclosure is 0.01 g/L-100 g/L, and is 5-20 g/L in an embodiment.

Compared with the prior art, the technical solutions of the present disclosure have the following significant advantages:
(1) It is discovered for the first time that sulfated gluco-galactooligosaccharide may be used as a drug for treating atopic dermatitis. It exerts a therapeutic effect by reducing the levels of inflammatory cytokines at the affected sites, and is safe and non-toxic with strong efficacy, thus exhibiting excellent pharmaceutical prospects.
(2) The sulfated gluco-galactooligosaccharide of the present disclosure has the effect of improving the skin condition of dinitrochlorobenzene (DNCB)-induced atopic dermatitis (AD) mice, and may improve skin conditions to achieve a therapeutic effect.
(3) Treatment with the sulfated gluco-galactooligosaccharide of the present disclosure may inhibit the aggregation and infiltration of mast cells in skin tissues.
(4) Treatment with the sulfated gluco-galactooligosaccharide of the present disclosure may reduce the impact of systemic immune responses in DNCB-induced AD mice, reducing changes in the weight of spleens and lymph nodes.
(5) The sulfated gluco-galactooligosaccharide of the present disclosure may inhibit the mRNA expression of inflammatory cytokines in an *in vitro* inflammatory cell model induced by TNF-α/IFN-γ.
(6) The sulfated gluco-galactooligosaccharide of the present disclosure exhibits good cytocompatibility.

### BRIEF DESCRIPTION OF THE DRAWINGS

FIG. 1 shows the effect of sulfated gluco-galactooligosaccharide on scratching behavior in DNCB-induced AD mice;
FIG. 2 shows the effect of sulfated gluco-galactooligosaccharide on weight changes in DNCB-induced AD mice;
FIGs. 3A-3B show the effect of sulfated gluco-galactooligosaccharide on ear and dorsal skin thickness changes in DNCB-induced AD mice; where FIG. 3A shows the effect of sulfated gluco-galactooligosaccharide on dorsal skin thickness changes in DNCB-induced AD mice, and FIG. 3B shows the effect of sulfated gluco-galactooligosaccharide on ear skin thickness changes in DNCB-induced AD mice;
FIG. 4 shows the effect of sulfated gluco-galactooligosaccharide on inflammatory lesions of dorsal skin in DNCB-induced AD mice;
FIGs. 5A-5B show representative H&E staining images of ear and dorsal skin with AD-like lesions in each group; where FIG. 5A shows representative H&E staining images of dorsal skin with AD-like lesions in each group, and FIG. 5B shows representative H&E staining images of ear skin with AD-like lesions in each group;
FIGs. 6A-6B show the effect of sulfated gluco-galactooligosaccharide on TBO staining of skin tissues in DNCB-induced AD mice; where FIG. 6A shows representative images of pathological features of dorsal skin tissues stained with toluidine blue, with black arrows indicating mast cells, and FIG. 6B shows the comparison of the number of mast cells;
FIGs. 7A-7D show the effect of sulfated gluco-galactooligosaccharide on mRNA expression of inflammatory cytokines in dorsal skin tissues of DNCB-induced AD mice;
FIGs. 8A-8D show the effect of sulfated gluco-galactooligosaccharide on mRNA expression of inflammatory cytokines in ear of DNCB-induced AD mice;
FIGs. 9A-9B show the effect of sulfated gluco-galactooligosaccharide on serum inflammatory cytokines IL-4 and IgE in DNCB-induced AD mice; where FIG. 9A shows the effect of sulfated gluco-galactooligosaccharide on serum inflammatory cytokine IgE in DNCB-induced AD mice, and FIG. 9B shows the effect of sulfated gluco-galactooligosaccharide on serum inflammatory cytokine IL-4 in DNCB-induced AD mice;
FIGs. 10A-10B show the effect of sulfated gluco-galactooligosaccharide on the weight of peripheral immune organs in DNCB-induced AD mice; where FIG. 10A shows the effect of sulfated gluco-galactooligosaccharide on spleen weight in DNCB-induced AD mice, and FIG. 10B shows the effect of sulfated gluco-galactooligosaccharide on lymph node weight in DNCB-induced AD mice; and
FIGs. 11A-11C show the effect of sulfated gluco-galactooligosaccharide on mRNA expression in HaCaT cells stimulated by TNF-α/IFN-γ.

### DETAILED DESCRIPTION OF THE EMBODIMENTS

The methods used in the following examples, if not indicated specifically, are all conventional methods in the art. The raw materials used in the following examples, without special instructions, are all commercially available. Unless otherwise defined, all technical and scientific terms used in the present disclosure have the meanings commonly understood by those of ordinary skill in the art to which this disclosure belongs.

The sulfated gluco-galactooligosaccharide in the present disclosure is prepared by conventional methods known in the art. Specifically, it may be prepared according to the methods taught in Yu N's article (Yu N, Fang R, Ding Z, et al. Preparation and Structural Characterization of a Sulfated Octasaccharide with Heparin-like Anticoagulant Activity [J]. Carbohydrate Polymers, 2025, 347:122782) and patent application CN 114533756 A.

In the examples described below, the sulfated gluco-galactooligosaccharide used has an average of 1.8 sulfate groups per sugar residue, *i.e.,* a degree of substitution of 1.8.

The present disclosure will be described in further detail below with reference to examples and the accompanying drawings.

### Example 1

Study on the improvement of AD mice model by sulfated gluco-galactooligosaccharide

### (1) Establishment of animal model

Female BALB/c mice (6-8 weeks old) provided by the Comparative Medicine Center of Yangzhou University were used in the experiment. All mice were housed under standard feeding conditions with a 12-hour light-dark cycle, and food and water were freely accessible to them. To establish the atopic dermatitis mouse model, the hair on the back of the mice was removed using a shaver and depilatory cream on Day 0 of the experiment. On Day 1 of the experiment, 100 µL of 20 g/L DNCB was applied to the back and 10 µL to the ear of each mouse to induce atopic dermatitis. On Day 5, 8 and 11 of the experiment, 100 µL of 5 g/L DNCB was applied to the back, 10 µL to the ear of each mouse every morning to challenge atopic dermatitis. The mice were euthanized 24 hours after the final challenge.

### (2) Experiment grouping

5 days after the establishment of the atopic dermatitis model, the mice were randomly divided into 4 groups (6 mice per group): a) normal control group (Ctrl group); b) model group (DNCB group), where 100 µL of normal saline was applied to the back and 10 µL to the ear of each mouse every day; c) low-dose group (DNCB+LSRO group), where 100 µL of 5 g/L aqueous solution of sulfated gluco-galactooligosaccharide was applied to the back of each mouse every day, and 10 µL of 5 g/L aqueous solution of sulfated gluco-galactooligosaccharide was applied to the ear of each mouse every day; and d) high-dose group (DNCB+HSRO group), where 100 µL of 20 g/L aqueous solution of sulfated gluco-galactooligosaccharide was applied to the back of each mouse every day, and 10 µL of 20 g/L aqueous solution of sulfated gluco-galactooligosaccharide was applied to the ear of each mouse every day. The skin condition of the mice was recorded daily.

### (3) Recording of ear and dorsal skin condition and mouse body weight

The skin of the mice was photographed at the same time every day to record the skin condition, and the body weight of the mice was measured.

### (4) Measurement of ear and dorsal skin thickness

The ear thickness was accurately measured every day using an electronic caliper. Before the mice were euthanized, the thickness of the dorsal skin was measured with a caliper. Three measurements were taken at different positions on the mid-back, near the neck, and near the tail, and the average value was taken as the final measurement result.

### (5) Scratching behavior test

The mice were placed in cages with sufficient space to exhibit various natural behaviors. Prior to the start of the experiment, the mice were allowed to acclimatize to the cages for 1 hour to ensure they could relax and display normal behavioral patterns. During this period, the behavior of the mice scratching the skin of their neck, ears and back with their paws was closely observed and recorded.

### (6) Skin histology analysis

The excised skin tissues were immediately fixed in 4% paraformaldehyde (prepared in PBS buffer) and embedded in paraffin. The sections were stained with hematoxylin and eosin (H&E) and toluidine blue (TBO) for histological analysis. Skin conditions were observed under a microscope.

### (7) Weight of spleens and lymph nodes in mice of each group

After the mice were euthanized, the spleens and lymph nodes of the mice in each group were harvested and weighed.

### (8) Determination of IgE and IL-4 in serum

Before euthanasia, the mice were anesthetized, and blood samples were collected from the orbit. The blood samples of the mice in each group were analyzed using a fully automatic hematology analyzer. After whole blood collection, the samples were allowed to clot naturally at room temperature, followed by centrifugation at 2000 r/min for 10 min. The supernatant was collected for the determination of IgE and IL-4 levels. The levels of IgE and IL-4 were measured using a double-antibody sandwich enzyme-linked immunosorbent assay, according to the manufacturer's instructions.

### (9) Gene expression analysis

After all the mice were euthanized, an appropriate amount of ear and dorsal skin tissues was harvested. RNA was extracted from the tissues using the liquid nitrogen grinding method. The extracted RNA was then reverse-transcribed into cDNA by means of reverse transcription. Real-time fluorescent quantitative PCR (qRT-PCR) was performed to specifically detect the expression of IL-1β, IL-4, IL-8 and TSLP.

These interleukin cytokines of IL-1β, IL-4 and IL-8 play important roles in inflammatory responses and immune mediation. TSLP may activate dendritic cell-mediated Th2 responses and exhibits high expression in atopic dermatitis skin lesions.

### (10) Data analysis

Statistical analysis was performed using One-way/Two-way ANOVA for comparisons between multiple groups. P < 0.05 was considered to indicate a statistically significant difference.

### (11) Result analysis

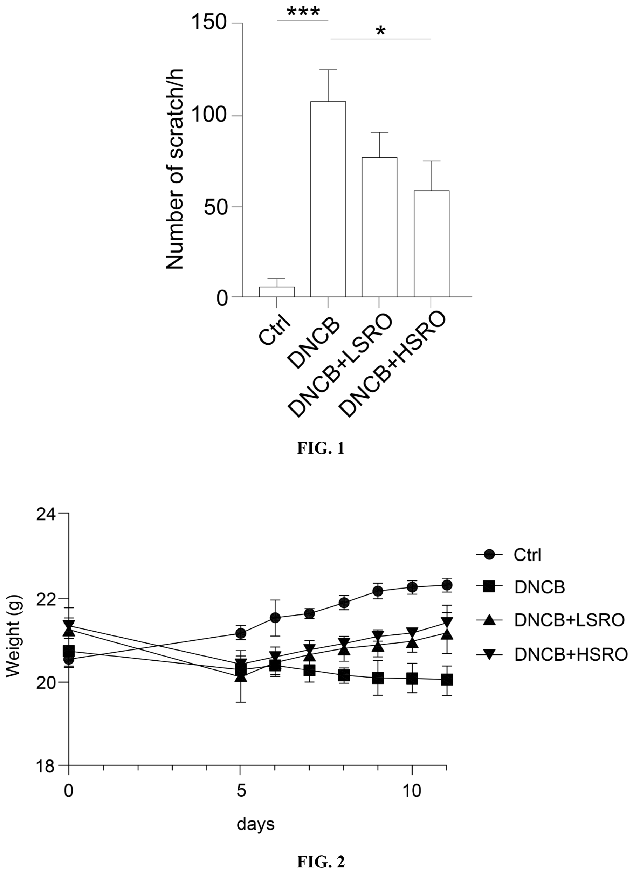

As shown in FIG. 1, sulfated gluco-galactooligosaccharide exerted a certain therapeutic effect on pruritus symptoms induced by DNCB and reduced scratching behavior in mice. The average scratching frequency was 107 times in the DNCB group, compared with 76 times in DNCB+LSRO group and only 58 times in DNCB+HSRO group. The scratching behavior was significantly reduced, indicating that skin pruritus was alleviated to a certain extent and the risk of skin damage was lowered.

As shown in FIG. 2, sulfated gluco-galactooligosaccharide exerted a certain protective effect against DNCB-induced weight loss in mice. The body weight of mice in the DNCB group and each treatment group slightly decreased compared with those in the Ctrl group. With continuous DNCB challenge, the body weight of mice in the DNCB group decreased significantly, whereas the weight loss in mice of the different-dose sulfated gluco-galactooligosaccharide treatment groups was not obvious.

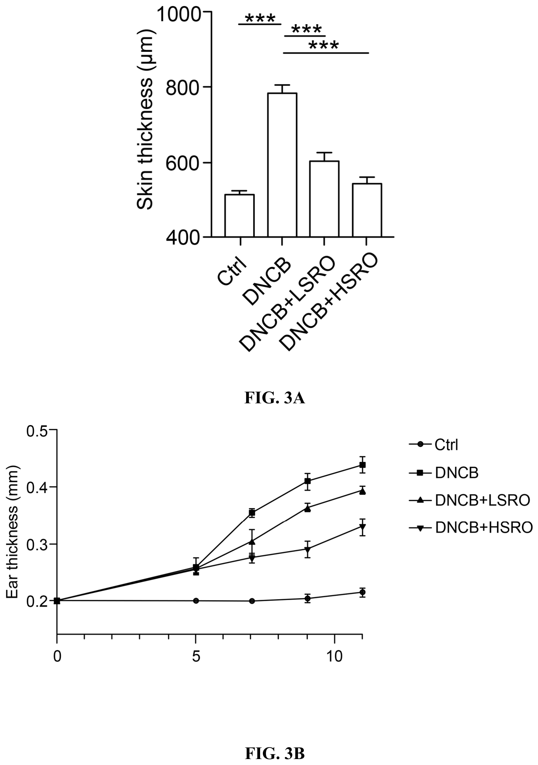

As shown in FIGs. 3A-3B, sulfated gluco-galactooligosaccharide alleviated DNCB-induced ear and dorsal skin thickening in mice. The average dorsal skin thickness was 785 µm in the DNCB group, compared with 606 µm in DNCB+LSRO group and 545 µm in DNCB+HSRO group. The average ear skin thickness was 0.44 mm in the DNCB group, compared with 0.375 mm in DNCB+LSRO group and 0.33 mm in DNCB+HSRO group. The skin thickness was significantly reduced, indicating that sulfated gluco-galactooligosaccharide alleviated DNCB-induced skin damage.

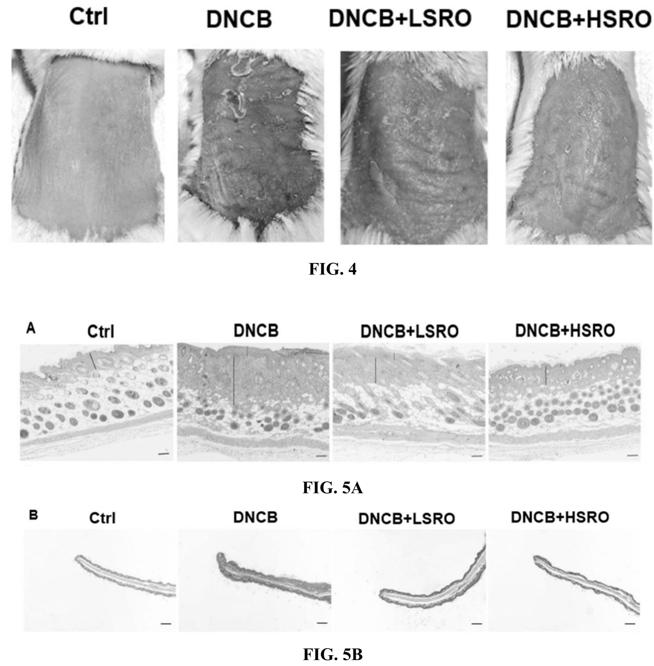

As shown in FIG. 4, sulfated gluco-galactooligosaccharide alleviated DNCB-induced dorsal skin inflammatory lesions in mice. DNCB was able to induce atopic dermatitis-like symptoms in the animal model, such as erythema, edema, papules and other inflammatory manifestations. After repeated topical application of DNCB, the dorsal skin of the mice exhibited AD clinical features such as dryness, erythema, erosion and edema. Compared with the DNCB group, the sulfated gluco-galactooligosaccharide treatment groups effectively alleviated these symptoms.

As shown in FIGs. 5A-5B, H&E staining results demonstrated that mice in the DNCB group exhibited symptoms such as epidermal hyperplasia, hyperkeratosis and inflammatory cell infiltration. Sulfated gluco-galactooligosaccharide maintained the skin structure integrity, alleviated skin lesions and protected skin tissues from damage (red lines in the figure indicated the dermal thickness, and the blue line indicated the epidermal thickness).

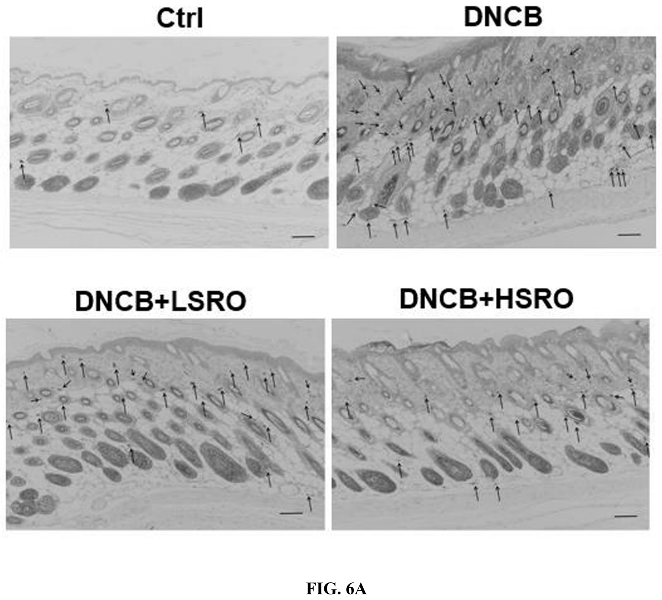

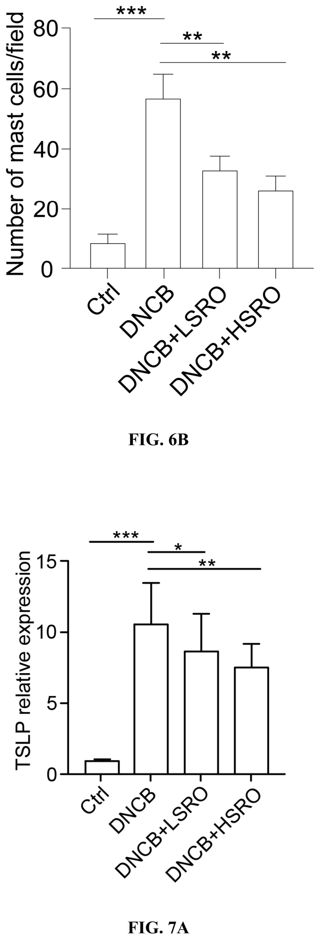

As shown in FIGs. 6A-6B, TBO staining results indicated that DNCB induced the infiltration of inflammatory cells such as mast cells into the skin tissues, whereas sulfated gluco-galactooligosaccharide treatment inhibited the aggregation and infiltration of mast cells in the skin tissues. The average number of mast cells was 56 in the DNCB group, compared with 32 in DNCB+LSRO group and 26 in DNCB+HSRO group. The number of mast cells was significantly reduced, indicating that sulfated gluco-galactooligosaccharide treatment could inhibit the aggregation and infiltration of mast cells in skin tissues.

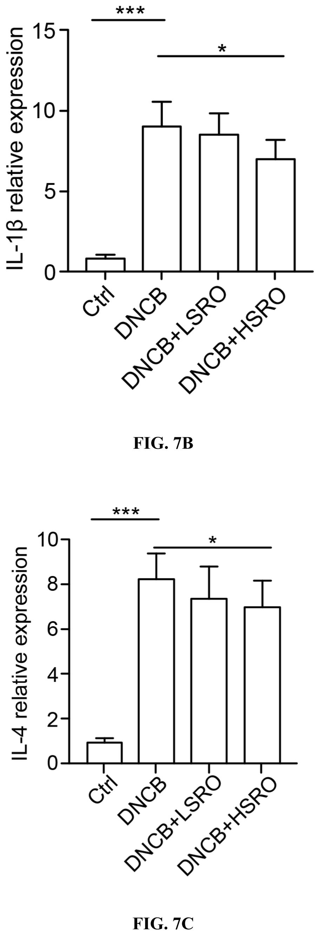

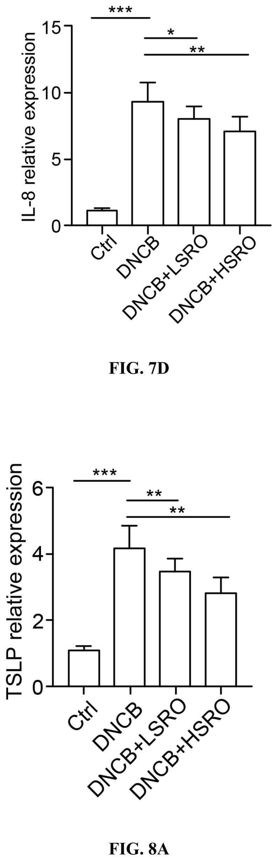

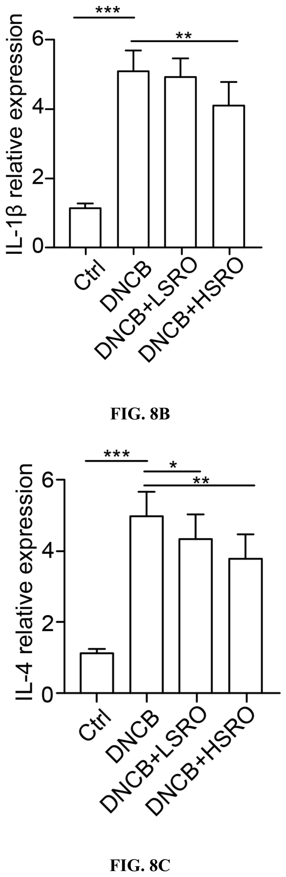

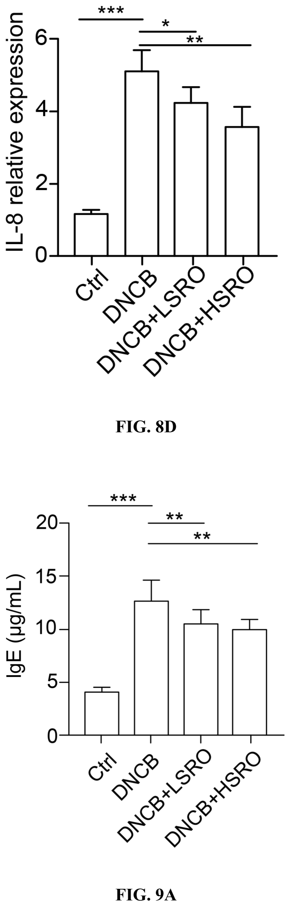

As shown in FIGs. 7A-7D and FIGs. 8A-8D, sulfated gluco-galactooligosaccharide significantly reduced the mRNA levels of IL-1β, IL-4, IL-8 and TSLP in the dorsal and ear skin tissues. Compared with the Ctrl group, the mRNA levels of IL-1β, IL-4, IL-8 and TSLP in the dorsal and ear skin tissues of the DNCB-induced AD mice (DNCB group) were significantly increased, whereas in the mice treated with sulfated gluco-galactooligosaccharide, the expression of these related cytokines was significantly decreased in a dose-dependent manner. These results indicated that the sulfated gluco-galactooligosaccharide could effectively inhibit the expression of these inflammatory cytokines, thereby alleviating the inflammatory response in AD mice.

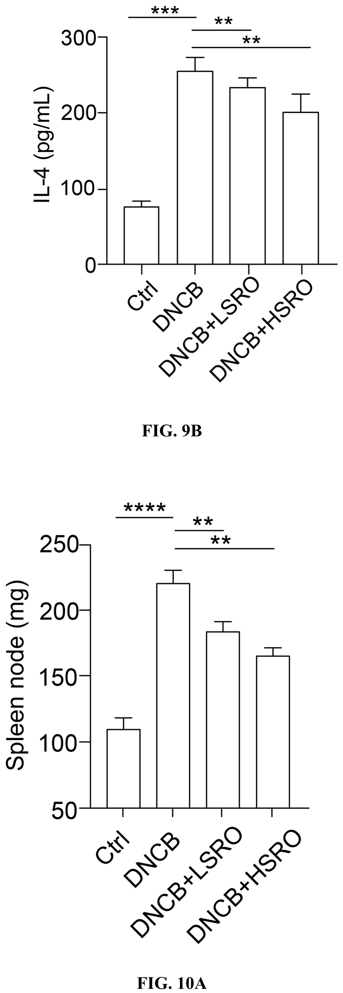

As shown in FIGs. 9A-9B, sulfated gluco-galactooligosaccharide reduced the protein levels of IL-4 and IgE in serum. The average level of IgE was 12.65 µg/mL in the DNCB group, compared with 10.45 µg/mL in DNCB+LSRO group and 10.01 µg/mL in DNCB+HSRO group. The average level of IL-4 was 253.5 pg/mL in the DNCB group, compared with 232.5 pg/mL in DNCB+LSRO group and 201.4 pg/mL in DNCB+HSRO group.

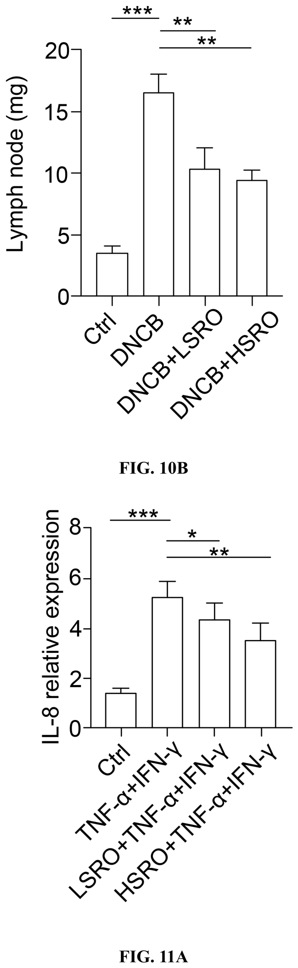

As shown in FIGs. 10A-10B, sulfated gluco-galactooligosaccharide reduced the impact of systemic immune responses in DNCB-induced AD mice, as evidenced by the reduction in spleen and lymph node weight gain. The average weight of the spleen was 221 mg in the DNCB group, compared with 183 mg in DNCB+LSRO group and 165 mg in DNCB+HSRO group. The average weight of the lymph nodes was 16.4 mg in the DNCB group, compared with 10.3 mg in DNCB+LSRO group and 9.45 mg in DNCB+HSRO group.

### Example 2

Effect of sulfated gluco-galactooligosaccharide on human immortalized keratinocytes (HaCaT)

### (1) Cell culture

HaCaT cells were used in the experiment, and their culture was carried out in high-glucose DMEM medium containing 10% fetal bovine serum, 1% streptomycin, and 1% erythromycin. The cells were incubated in a carbon dioxide incubator at 37°C and passaged using trypsin digestion.

### (2) Model establishment

1) HaCaT cells were uniformly seeded in 6-well plates at a density of 5 × 10⁵ cells per well. Control group and experimental groups were set up, namely the Ctrl group, TNF-α+IFN-γ group, LSRO+TNF-α+IFN-γ group and HSRO+TNF-α+IFN-γ group.
2) TNF-α/IFN-γ (10 ng/mL) was added to the TNF-α+1FN-γ, LSRO+TNF-α+IFN-γ and HSRO+TNF-α+IFN-γ groups, respectively, while an equal volume of PBS solution (pH = 7.2) was added to the Ctrl group. The plates were shaken crosswise to mix thoroughly, followed by incubation at 37°C for 18 h in a carbon dioxide incubator.
3) PBS, PBS, 100 µg/mL and 200 µg/mL sulfated gluco-galactooligosaccharide were added to the Ctrl, TNF-α+IFN-γ, LSRO+TNF-α+IFN-γ and HSRO+TNF-α+IFN-γ groups, respectively. The 6-well plates were placed in a carbon dioxide incubator at 37°C, and the cells were harvested after 6 h of incubation.

### (3) Gene expression analysis

HaCaT cells were harvested, followed by RNA extraction and reverse transcription into cDNA. The expression levels of related genes such as IL-4, IL-6 and TNF-α were detected via fluorescent quantitative PCR.

### (4) Data analysis

Statistical analysis was performed using One-way ANOVA for comparisons between multiple groups. P < 0.05 was considered to indicate a statistically significant difference.

### (5) Result analysis

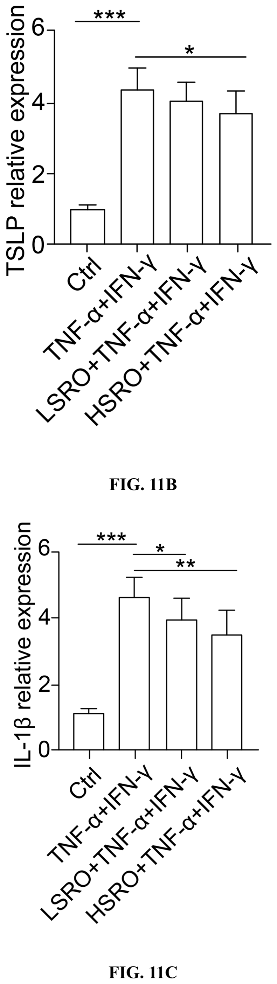

As shown in FIGs. 11A-11C, in the TNF-α/IFN-γ-induced inflammatory model of keratinocytes *in vitro,* sulfated gluco-galactooligosaccharide could significantly inhibit the elevation of inflammatory cytokines induced by TNF-α/IFN-γ, which further indicated that it exerted potent anti-inflammatory effects in *in vitro* model.

## Claims

1. Sulfated gluco-galactooligosaccharide for use in treating atopic dermatitis, wherein the sulfated gluco-galactooligosaccharide has a structural formula: 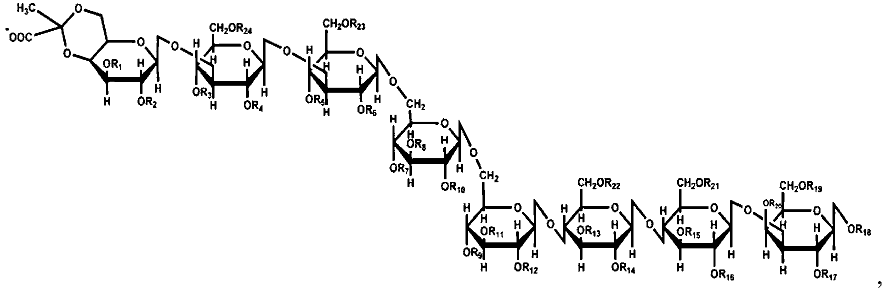 and wherein, R₁, R₂, R₃, R₄, R₅, R₆, R₇, R₈, R₉, R₁₀, R₁₁, R₁₂, R₁₃, R₁₄, R₁₅, R₁₆, R₁₇, R₁₈, R₁₉, R₂₀, R₂₁, R₂₂, R₂₃ and R₂₄ are independently SO₃⁻ or H, but are not all H.

2. The sulfated gluco-galactooligosaccharide for use in according to claim 1, wherein in the sulfated gluco-galactooligosaccharide, an average number of sulfate groups per sugar residue is 1.8.

3. The sulfated gluco-galactooligosaccharide for use in according to claim 1, wherein a dosage form of the sulfated gluco-galactooligosaccharide for treating atopic dermatitis is a topical solution or a cream.

4. The sulfated gluco-galactooligosaccharide for use in according to claim 1, wherein a route of administration of the sulfated gluco-galactooligosaccharide for treating atopic dermatitis is topical application.

5. The sulfated gluco-galactooligosaccharide for use in according to claim 1, wherein a subject of administration of the sulfated gluco-galactooligosaccharide for treating atopic dermatitis is a human or non-human animal that may develop or have developed atopic dermatitis.

6. The sulfated gluco-galactooligosaccharide for use in according to claim 1, wherein a concentration of the sulfated gluco-galactooligosaccharide in a drug for treating atopic dermatitis is 0.01 g/L to 100 g/L.

7. The sulfated gluco-galactooligosaccharide for use in according to claim 1, wherein a concentration of the sulfated gluco-galactooligosaccharide in a drug for treating atopic dermatitis is 5 g/L to 20 g/L.
